# EUROPÄISCHE PATENTSCHRIFT

(11) **EP 1 718 585 B1**
(45) Veröffentlichungstag und Bekanntmachung des Hinweises auf die Patenterteilung: **30.05.2012**
(21) Anmeldenummer: 05701352.6
(22) Anmeldetag: 04.02.2005
(51) Int. Cl.: C07C 7/08, C07C 11/167

(54) **VERFAHREN ZUR GEWINNUNG VON ROH-1,3-BUTADIEN**
METHOD FOR OBTAINING RAW-1,3-BUTADIENE
PROCEDE POUR RECUPERER DU 1,3-BUTADIENE BRUT

(30) Priorität: 06.02.2004 DE 102004005930
(43) Veröffentlichungstag der Anmeldung: 08.11.2006
(73) Patentinhaber: BASF SE, 67056 Ludwigshafen (DE)
(72) Erfinder: HEIDA, Bernd, 67158 Ellerstadt (DE)
(86) Internationale Anmeldenummer: PCT/EP2005/001152
(87) Internationale Veröffentlichungsnummer: WO 2005/075388

(56) Entgegenhaltungen:
- WO-A-02/062733
- WO-A-2004/011407
- US-A1- 2003 181 772

## Beschreibung

Die Erfindung betrifft ein Verfahren zur Gewinnung von Roh-1,3-Butadien du rch Extraktivdestillation mit einem selektiven Lösungsmittel aus einem C₄-Schnitt, enthaltend C₄-Acetylene als Nebenkomponenten in einer Trennwandkolonne oder in thermisch gekoppelten Kolonnen.

Die Gewinnung von 1,3-Butadien aus einem C₄-Schnitt ist wegen der geringen Unterschiede der Komponenten des C₄-Schnittes in den relativen Flüchtigkeiten ein kompliziertes destillationstechnisches Problem. Daher wird die Auftrennung durch eine so genannte Extraktivdestillation durchgeführt, das heißt eine Destillation unter Zugabe eines Extraktionsmittels, das einen höheren Siedepunkt als das aufzutrennende Gemisch aufweist und das die Unterschiede in den relativen Flüchtigkeiten der aufzutrennenden Komponenten erhöht. Durch Einsatz geeigneter Extraktionsmittel kann aus dem genannten C₄-Schnitt mittels Extraktivdestillation eine Roh-1,3-Butadien-Fraktion erhalten werden, die anschließend in Reindestillationskolonnen weiter gereinigt wird.

Vorliegend wird als Roh-1,3-Butadien ein Kohlenwasserstoffgemisch bezeichnet, das aus einem C₄-Schnitt gewonnen wurde, aus dem mindestens 90 Gew.-% der Summe aus Butanen und Butenen, bevorzugt mindestens 98 Gew.-% der Summe aus Butanen und Butenen, besonders bevorzugt mindestens 99 Gew.-% der Summe aus Butanen und Butenen und gleichzeitig mindestens 90 Gew.-% der C₄-Acetylene, bevorzugt mindestens 96 Gew.-% der C₄-Acetylene, besonders bevorzugt mindestens 99 Gew.-% der C₄-Acetylene, abgetrennt wurden. Roh-1,3-Butadien enthält das Wertprodukt 1,3-Butadien häufig in einem Anteil von mindestens 80 Gew.-%, bevorzugt 90 Gew.-%, besonders bevorzugt mehr als 95 Gew.-%, Rest Verunreinigungen.

Dem gegenüber wird als Rein-1,3-Butadien ein Kohlenwasserstoffgemisch bezeichnet, das das Wertprodukt 1,3-Butadien in einem Anteil von mindestens 98 Gew₋-%, bevorzugt von mindestens 99,5 Gew.-%, besonders bevorzugt im Bereich zwischen 99,7 und 99,9 Gew.-%, Rest Verunreinigungen, enthält.

Aus DE-A 101 05 660 ist ein Verfahren mit vereinfachter konstruktiver Ausgestaltung der Apparate gegenüber älteren Verfahren bekannt: die Auftrennung des C₄-Schnitts erfolgt in einer Trennwandkolonne mit bis zum oberen Ende der Trennwandkolonne durchgezogener Trennwand und einer der Trennwandkolonne vorgeschalteten Extraktivwaschkolonne. Nach dem Verfahren der DE-A 101 05 660 wird aus dem Sumpf der zur Extraktivdestillation eingesetzten Trennwandkolonne ein teilentgaster Lösungsmittelstrom abgezogen. Der Begriff "teilentgastes Lösungsmittel" ist hierbei dem in der Extraktivdestillation zur Gewinnung von 1,3-Butadien tätigen Fachmann bekannt und bezeichnet ein selektives Lösungsmittel, das noch gelöste Komponenten aus dem aufzutrennenden C₄-Schnitt enthält und zwar die Komponenten, die die größte Affinität zum selektiven Lösungsmittel aufweisen. Hierzu zählen insbesondere die C₄-Acetylene, vor allem Ethylacetylen und Vinylacetylen.

Ein lediglich teilentgaster Lösungsmittelstrom kann jedoch nicht in die Extraktivdestillation recycliert werden, da sich ansonsten die spezifikationsschädlichen Acetylene aufpegeln würden. Daher musste der aus der Trennwandkolonne abgezogene Sumpfstrom vor der Recyclierung in die Extraktivdestillation zunächst einer Ausgaserkolonne, wie sie zum Beispiel aus DE-A 27 24 365 bekannt ist, zugeführt werden, die bei niedrigerem Druck gegenüber der Kolonne, aus deren Sumpf der teilentgaste Strom abgezogen wird, betrieben wird. In der Ausgaserkolonne erfolgt die Aufbereitung des teilentgasten Lösungsmittelstromes unter Erhalt eines gereinigten, das heißt vollständig entgasten Lösungsmittels am Sumpf und eines gasförmigen Kohlenwasserstromes am Kopf der Ausgaserkolonne, der über einen Kompressor in den unteren Bereich der Extraktivdestillationskolonne zurückgeführt wird. Die Acetylen werden über einen Seitenstrom ausgeschleust.

Nach dem Verfahren der DE-A 27 24 365 enthält jedoch der aus der Trennwandkolonne abgezogene Sumpfstrom, der der Ausgaserkolonne zugeführt wird, neben den C₄-Acetylenen auch noch beträchtliche Mengen des Wertproduktes 1,3-Butadien. Das 1,3-Butadien geht in den Kopfstrom der Ausgaserkolonne, der, bei einer wirtschaftlichen Betriebsweise, nicht verworfen werden kann, sondern über einen Kompressor in Extraktivdestillation zurückgeführt wird, die bei höherem Druck gegenüber dem Ausgaser, betrieben wird. Der Kompressor hat einen hohen Energieverbrauch, daher war auch das Verfahren der DE-A 27 24 365 bereits ein Fortschritt gegenüber älteren Verfahren, wonach Kompressoren mit dreifachem Energieverbrauch erforderlich waren. Zum Anmeldezeitpunkt der DE-A 27 24 365 war es dem Fachmann jedoch nicht bekannt, dass eine Verfahrensführung, die auf den Kompressor vollständig verzichten kann, in technisch einfacher Weise realisiert werden kann.

In der DE-A 103 22 655 ist ein Verfahren beschrieben, wonach über die Regelung des Energieeintrags in die Trennwandkolonne über den Sumpfverdampfer desselben sowie die Auslegung der Zahl der theoretischen Trennstufen im unteren gemeinsamen Kolonnenbereich die Fahrweise der Trennwandkolonne so eingestellt wird, dass man aus der Trennwandkolonne einen Sumpfstrom abziehen kann, der bereits gereinigtes Lösungsmittel enthält.

US 2003/0181772 offenbart einem process für die Aufarbeitung einen C4-Fraktion.

Entsprechend entfällt die Ausgaserkolonne sowie der Kompressor zur Rückführung von 1,3-Butadien enthaltendem Strom in die Extraktivdestillation.

Unter dem Begriff gereinigtes Lösungsmittel oder voll entgastes Lösungsmittel wird vorliegend ein Lösungsmittel verstanden, das so weit an Komponenten aus dem C₄-Schnitt abgereichert ist, dass es für den Einsatz als selektives Lösungsmittel zur Extraktivdestillation eines C₄-Schnitts geeignet ist, wobei die vorgegebenen Spezifikationen für Roh-1,3-Butadien und Raffinat 1 eingehalten werden. Schlüsselkomponenten sind hierbei C₄-Acetylene, insbesondere Ethylacetylen und Vinylacetylen.

Demgegenüber war es Aufgabe der Erfindung, ein verbessertes Verfahren zur Extraktivdestillation von 1,3-Rohbutadien in kompressorloser Fahrweise zur Verfügung zu stellen, das insbesondere weniger Fouling in den Kolonnen, eine erhöhte Betriebssicherheit und Wirtschaftlichkeit gewährleistet. Entsprechend wurde ein Verfahren zur Gewinnung von Roh-1,3-Butadien durch Extraktivdestillation mit einem selektiven Lösungsmittel aus einem C₄-Schnitt, enthaltend C₄-Acetylene als Nebenkomponenten gefunden, das man in einer Trennwandkolonne mit einem Sumpfverdampfer durchführt, in der eine Trennwand in Kolonnenlängsrichtung unter Ausbildung eines ersten Teilbereichs, eines zweiten Teilbereichs und eines unteren gemeinsamen Kolonnenbereichs angeordnet und der eine Extraktivwaschkolonne vorgeschaltet ist, das dadurch gekennzeichnet ist, dass man den Energieeintrag in die Trennwandkolonne über den Sumpfverdampfer so regelt, dass man aus der Trennwandkolonne einen Sumpfstrom abzieht, enthaltend mit den C₄-Acetylenen beladenes Lösungsmittel, dessen Anteil an 1,3-Butadien auf das maximal 0,1 bis 2-fache des Anteils an C4 Acetylenen so begrenzt ist, dass der Verlust an 1,3-Butadien wirtschaftlich akzeptabel ist, und dass man den Sumpfstrom einem Acetylene-Ausgaser zuführt und im Acetylene-Ausgaser die C₄-Acetylene über Kopf ausstrippt und gereinigtes Lösungsmittel als Sumpfstrom gewinnt.

Es wurde gefunden, dass es möglich ist, in der Trennwandkolonne den überwiegenden Anteil der Kohlenwasserstoffe aus dem C₄-Schnitt abzutrennen, so dass im selektiven Lösungsmittel im Wesentlichen nur die darin am besten löslichen Kohlenwasserstoffe, d.h. die C₄-Acetylene, verbleiben. Daher ist es lediglich erforderlich, aus dem Sumpfstrom der Trennwandkolonne die C₄-Acetylene abzutrennen, unter Erhalt eines gereinigten Lösungsmittels, das man vorteilhaft in die Extraktivdestillation rezykliert. Da der 1,3-Butadiengehalt im Sumpfstrom der Extraktivdestillationskolonne auf niedrige Werte abgesenkt werden kann, ist es wirtschaftlich vertretbar, dieselben nicht erneut in die Extraktivdestillation, unter energieintensivem Einsatz eines Kompressors, zurückzuführen.

Der vorliegend als Ausgangsgemisch einzusetzende so genannte C₄-Schnitt ist ein Gemisch von Kohlenwasserstoffen mit überwiegend vier Kohlenstoffatomen pro Molekül, C₄-Schnitte werden beispielsweise bei der Herstellung von Ethylen und/oder Propylen durch thermisches Spalten einer Petroleumfraktion wie verflüssigtes Petroleumgas, Leichtbenzin oder Gasöl erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an C₃- und C₆-Kohlenwasserstoffen, sowie Butine, insbesondere 1-Butin (Ethylacetylen) und Butenin(Vinylacetylen). Dabei beträgt der 1,3-Butadiengehalt im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Für die eingangs bereits definierte Extraktivdestillation kommen für das vorliegende Trennproblem, der Gewinnung von 1,3-Butadien aus dem C₄-Schnitt, als Extraktionsmittel, d.h. als selektive Lösungsmittel, generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt.

Geeignete selektive Lösungsmittel für das erfindungsgemäße Verfahren sind zum Beispiel Butyrolacton, Nitrile wie Acetonitril, Propionitril, Methoxypropioninitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon. Im allgemeinen werden N-alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Colösungsmitteln wie Wasser und/oder tert.-Butylether, zum Beispiel Methyl-tert.-butylether, Ethyl-tert.-butylether, Propyl-tert.-butylether, n- oder iso-Butyl-tert.-butylether eingesetzt werden.

Besonders geeignet ist N-Methylpyrrolidon, vorliegend abgekürzt als NMP bezeichnet, bevorzugt in wässriger Lösung, insbesondere mit 7 bis 10 Gew.-% Wasser, besonders bevorzugt mit 8,3 Gew.-% Wasser.

Die Extraktivdestillation wird in einer Trennwandkolonne durchgeführt, in der eine Trennwand in Kolonnenlängsrichtung und Ausbildung eines ersten Teilbereichs, eines zweiten Teilbereichs und eines unteren gemeinsamen Kolonnenbereichs angeordnet ist und die mit einer vorgeschalteten Extraktivwaschkolonne verbunden ist.

Trennwandkolonnen werden in bekannter Weise für komplexere Trennaufgaben, in der Regel für Gemische von mindestens drei Komponenten, wobei die Einzelkomponenten jeweils in reiner Form erhalten werden sollen, eingesetzt. Sie weisen eine Trennwand auf, d.h. in der Regel ein in Kolonnenlängsrichtung ausgerichtetes ebenes Blech, das eine Quervermischung der Flüssigkeits- und Brüdenströme in Teilbereichen der Kolonne verhindert.

Vorliegend wird eine besonders ausgestaltete Trennwandkolonne eingesetzt, deren Trennwand bis am obersten Punkt der Kolonne durchgezogen ist und somit eine Vermischung von Flüssigkeits- und Brüdenströmen lediglich im unteren gemeinsamen Kolonnenbereich gestattet. Der so genannte erste und zweite Teilbereich sind durch die Trennwand voneinander getrennt.

In der dem Fachmann bekannten Weise kann die Trennwandkolonne durch entsprechend verschalterte thermisch gekoppelte Kolonnen ersetzt werden.

Die Extraktivwaschkolonne ist eine Gegenstromwaschkolonne. In allen Kolonnen gibt es keine Einschränkungen bezüglich der einsetzbaren trennwirksamen Einbauten; aus Kostengründen sind Füllkörper bevorzugt.

Der Acetylene-Ausgaser ist eine Abtriebskolonne; der mit C₄-Acetlyenen beladene Sumpfstrom aus der Extraktivdestillation wird im oberen Bereich des Acetylene-Ausgasers aufgegeben und daraus werden im Gegenstrom mit dem aufsteigenden heißen Gasstrom die C₄-Acetylene ausgegast.

Auf den Acetylene-Ausgaser ist vorzugsweise ein Wasserwäscher aufgesetzt, worin Lösungsmittelreste aus dem ausgegasten Acetylene-Strom mit Rücklauf und Frischwasser ausgewaschen werden. Der Wasserwäscher ist vorzugsweise mit bedeutend geringerem Durchmesser gegenüber dem Acetylene-Ausgaser dimensioniert. Am Kopf des Wasserwäschers fällt ein die C₄-Acetylene enthaltender Strom an, der kondensiert und teilweise als Rücklauf wieder auf den Wasserwäscher aufgegeben und im übrigen aus dem Verfahren ausgeschleust, insbesondere einem Cracker zugeführt oder verbrannt wird.

Der Sumpfstrom aus dem Acetylene-Ausgaser enthält gereinigtes Lösungsmittel und wird bevorzugt in die Extraktivdestillation recycliert.

Nach einer bevorzugten Verfahrensführung wird
- der C₄-Schnitt dem ersten Teilbereich der Trennwandkolonne, bevorzugt in dessen mittleren Bereich, zugeführt,
- der Kopfstrom aus dem ersten Teilbereich der Trennwandkolonne der Extraktivwaschkolonne, in deren unteren Bereich, zugeführt,
- in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
- die im selektiven Lösungsmittel weniger als 1,3-Butadien löslichen Komponenten des C₄-Schnittes werden über Kopf der Extraktivwaschkolonne als so genanntes Raffinat 1 abgezogen,
- der Sumpfstrom aus der Extraktivwaschkolonne in den oberen Bereich des ersten Teilbereichs der Trennwandkolonne zurückgeführt,
- der Trennwandkolonne im oberen Bereich des zweiten Teilbereichs ein zweiter Teilstrom des selektiven Lösungsmittels zugeführt,
- das Kopfprodukt aus dem zweiten Teilbereich (B) der Trennwandkolonne als Roh-1,3-Butadien abgezogen und
- aus dem unteren gemeinsamen Kolonnenbereich der Trennwandkolonne ein Sumpfstrom bestehend aus mit den C₄-Acetylenen beladenem Lösungsmittel, dessen Anteil an 1,3-Butadien so begrenzt ist, dass der Verlust an 1,3-Butadien wirtschaftlich akzeptabel ist, abgezogen wird,
- der Sumpfstrom (17) dem Acetylene-Ausgaser (AG) zugeführt wird, worin die C₄-Acetylene über Kopf ausgestrippt und gereinigtes Lösungsmittel als Sumpfstrom (27) gewonnen wird, der in das Verfahren rezykliert wird.

Bevorzugt wird somit der aufzutrennende C₄-Schnitt dem ersten Teilbereich der Trennwandkolonne, besonders bevorzugt in dessen mittleren Bereich, zugeführt;
der Kopfstrom aus dem ersten Teilbereich der Trennwandkolonne wird der vorgeschalteten Extraktivwaschkolonne in deren unteren Bereich zugeführt,
in der Extraktivwaschkolonne eine Gegenstromextraktion durch Beaufschlagung mit einem ersten Teilstrom des selektiven Lösungsmittels im oberen Bereich der Extraktivwaschkolonne durchgeführt,
die im selektiven Lösungsmittel weniger als 1,3-Butadien-löslichen Komponenten des C₄-Schnittes über Kopf der Extraktivwaschkolonne abgezogen, besonders bevorzugt in einem Kondensator am Kopf der Extraktivwaschkolonne kondensiert und teilweise als Rücklauf erneut auf die Extraktivwaschkolonne aufgegeben, im übrigen als ein überwiegend Butane und Butene enthaltendes Nebenprodukt, häufig als Raffinat 1 bezeichnet, abgezogen.

Durch die Zuführung des Sumpfstroms der Extraktivwaschkolonne, d.h. eines Stromes, der neben dem selektiven Lösungsmittel 1,3-Butadien, Butane, Butene sowie die besser als 1,3-Butadien im selektiven Lösungsmittel löslichen Komponenten des C₄-Schnitts enthält, in den oberen Bereich des ersten Teilbereichs der Trennwandkolonne kann durch den Stoffaustausch zwischen diesem Strom und dem dampfförmig aufgegebenen C₄-Schnitt im oberen Bereich des ersten Teilbereichs der Trennwandkolonne eine Gegenstromextraktion unter Abreicherung der im selektiven Lösungsmittel weniger als das 1,3-Butadien löslichen Komponenten am Kopf des ersten Teilbereichs der Trennwandkolonne erfolgen.

Am unteren Ende der Trennwand fällt ein dampfförmiger Strom an, der neben 1,3-Butadien die im selektiven Lösungsmittel besser als 1,3-Butadien-löslichen Komponenten des C₄-Schnitts, insbesondere C₄-Acetylene, enthält. Diese werden aus dem aufsteigenden dampfförmigen Strom im Gegenstrom mit einem zweiten Teilstrom des selektiven Lösungsmittels, das im oberen Bereich des zweiten Teilbereichs der Trennwandkolonne aufgegeben wird, ausgewaschen. Das dampfförmige Kopfprodukt aus dem zweiten Teilbereich der Trennwandkolonne wird abgezogen, wobei es bevorzugt in einem Kondensator am Kolonnenkopf kondensiert, ein Teilstrom des kondensierten Kopfstromes zurück auf den Teilbereich B der Trennwandkolonne als Rücklauf gegeben und der kondensierte Kopfstrom wird im übrigen als Roh-1,3-Butadien abgezogen wird.

Im unteren gemeinsamen Kolonnenbereich erfolgt eine Entgasung des Lösungsmittels, wobei am Sumpf der Extraktivdestillationskolonne ein Lösungsmittel erhalten wird, das die C₄-Acetylene enthält und 1,3-Butadien in einem Anteil, dessen Verlust wirtschaftlich akzeptabel ist.

Bei der Bestimmung des hierfür erforderlichen Energieeintrags über den Sumpfverdampfer der Extraktivdestillationskolonne wird der Verfahrenstechniker die thermische Belastbarkeit der Substanz oder des Substanzgemisches berücksichtigen, die jeweils im konkreten Fall als selektives Lösungsmittel eingesetzt wurden.

Sofern es die thermische Belastbarkeit des selektiven Lösungsmittels zulässt, wird die Temperatur im Sumpf der Extraktivdestillationskolonne vorteilhaft so hoch eingestellt, dass am Kopf der Extraktivdestillationskolonne noch mit preiswerten Kühlmitteln, beispielsweise mit Flusswasser kondensiert werden kann.

Ist die thermische Belastbarkeit des konkret eingesetzten selektiven Lösungsmittels jedoch bei der Temperatur, die notwendig wäre, um am Sumpf Lösungsmittel zu erhalten, dessen Anteil an 1,3-Butadien so begrenzt ist, dass der Verlust an 1,3-Butadien wirtschaftlich akzeptabel ist, nicht ausreichend, muss bei einer Temperatur am Kolonnensumpf gearbeitet werden, die für das selektive Lösungsmittel noch zulässig ist und entsprechend am Kolonnenkopf mit einem aufwändigeren Kühlmittel gegenüber Flusswasser gekühlt werden.

Ein besonders bevorzugtes selektives Lösungsmittel ist, wie vorstehend ausgeführt, NMP, bevorzugt in wässriger Lösung, insbesondere mit 7 bis 10 Gew.-% Wasser, besonders bevorzugt mit 8,3 Gew.-% Wasser.

Unter der Voraussetzung, dass NMP als selektives Lösungsmittel eingesetzt wird, wird die Temperatur im Sumpfverdampfer der Extraktivdestillationskolonne bevorzugt im Bereich zwischen 150 und 210°C, besonders bevorzugt bei 178°C, eingestellt. Entsprechend wird der Kopfdruck im zweiten Teilbereich der als Trennwandkolonne ausgestalteten Extraktivdestillationskolonne im Bereich von 1 bis 10 bar absolut, bevorzugt von 2 bis 5 bar absolut, besonders bevorzugt bei 3,5 bar absolut, eingestellt. Bevorzugt wird der Acetylene-Ausgaser bei einem Kopfdruck im Bereich von 1 bar absolut bis maximal dem Sumpfdruck in der Trennwandkolonne (TK), betrieben.

Es ist grundsätzlich nicht erforderlich, die Gewinnung des Nebenprodukts aus Butanen und Butenen, des so genannten Raffinats 1, in einer von der Extraktivdestillationskolonne getrennten, vorgeschalteten Extraktivwaschkolonne vorzusehen. Es ist auch möglich, die Extraktivwaschkolonne in den ersten Teilbereich der als Extraktivdestillationskolonne eingesetzten Trennwandkolonne zu integrieren, sofern es technisch und wirtschaftlich unter Berücksichtigung der konkreten Rahmenbedingungen für das Verfahren, insbesondere der Zusammensetzung des aufzutrennenden C₄-Schnitts und der Spezifikation für Raffinat 1 realisierbar ist, die Zahl der theoretischen Trennstufen im ersten Teilbereich der Trennwandkolonne entsprechend zu erhöhen.

Die nachstehend beschriebenen bevorzugten Verfahrensvarianten aus dem Verfahren der DE-A 101 05 660 sind auch für das Verfahren der vorliegenden Erfindung gleichermaßen anwendbar:

In einer bevorzugten Verfahrensvariante wird der Brüdenstrom am unteren Ende der Trennwand der Trennwandkolonne durch geeignete Maßnahmen dergestalt aufgetrennt, dass der Teilstrom, der in den ersten Teilbereich der Trennwandkolonne geleitet wird, größer ist als der Teilstrom, der in den zweiten Teilbereich der Trennwandkolonne geleitet wird. Durch die Regelung der Aufteilung des Brüdenstroms am unteren Ende der Trennwand kann in einfacher und zuverlässiger Weise die geforderte Produktspezifikation des am Kopf des zweiten Teilbereichs der Trennwandkolonne abgezogenen Roh-1,3-Butadien-Stroms gewährleistet werden.

Besonders bevorzugt kann eine derartige ungleiche Auftrennung des Brüdenstroms am unteren Ende der Trennwand dadurch erreicht werden, dass die Trennwand außermittig angeordnet wird, und zwar der Gestalt, dass der zweite Teilbereich kleiner ist gegenüber dem ersten Teilbereich der Trennwandkolonne.

Besonders bevorzugt wird die Trennwand außermittig dergestalt angeordnet, dass das Querschnittsverhältnis des ersten Teilbereichs zum zweiten Teilbereich im Bereich von 8:1 bis 1,5:1, insbesondere bei 2,3:1 liegt.

Alternativ oder zusätzlich zur außermittigen Anordnung der Trennwand kann der Brüdenstrom am unteren Ende der Trennwand durch weitere Maßnahmen, beispielsweise Klappen oder Leitbleche im gewünschten Verhältnis auf die beiden Teilbereiche der Trennwandkolonne aufgeteilt werden.

Eine weitere zusätzliche oder alternative Maßnahme zur Aufteilung des Brüdenstroms am unteren Ende der Trennwand besteht in der Einstellung der Wärmeabführungsleistung des Kondensators am Kopf des zweiten Teilbereichs der Trennwandkolonne.

Eine bevorzugte Verfahrensvariante ist dadurch gekennzeichnet, dass die Drücke am oberen Ende der beiden Teilbereiche der Trennwandkolonne jeweils getrennt regelbar sind. Dadurch kann die erforderliche Produktspezifikation des Roh-1,3-Butadiens sichergestellt werden.

Die Kopfdrücke der beiden Teilbereiche der Trennwandkolonne können bevorzugt jeweils über eine Split-Range-Regelung eingestellt werden. Der Begriff Split-Range-Regelung bezeichnet in bekannter Weise eine Anordnung, wonach der Druckreglerausgang gleichzeitig mit der Inertgas- und der Entlüftungsleitung verbunden ist. Der Ventilstellbereich des Druckreglers ist so geteilt, dass jeweils nur ein Ventil betätigt wird, d.h. entweder es strömt Inertgas zu oder es wird entlüftet. Dadurch können die Inertgasmenge wie auch die mit dem Abluftstrom verbundenen Produktverluste minimiert werden.

Zusätzlich oder alternativ zur Split-Range-Regelung ist es möglich, die Kopfdrücke in den beiden Teilbereichen der Trennwandkolonne jeweils über die Wärmeabführleistung der Kondensatoren am Kopf des zweiten Teilbereichs der Trennwandkolonne bzw. am Kopf der Extraktivwaschkolonne zu regeln.

In einer bevorzugten Ausführungsvariante ist es möglich, den Acetylene-Ausgaser konstruktiv in den unteren Bereich der Trennwandkolonne zu integrieren. Hierfür muss die Zahl der theoretischen Trennstufen im unteren gemeinsamen Kolonnenbereich der Trennwandkolonne entsprechend erhöht werden und es muss an der Stelle, die dem oberen Ende des Acetylene-Ausgasers entspricht, eine gasdichte Abtrennung in der Trennwandkolonne vorgesehen werden, wobei jedoch in geeigneter Weise eine Flüssigkeitsverbindung, beispielsweise durch Abziehen der Flüssigkeit oberhalb der Abtrennung und erneute Zuführung derselben unterhalb der Abtrennung, gewährleistet wird.

Der Wärmeinhalt des Sumpfstromes aus der Extraktivdestillationskolonne kann vorteilhaft zur Wärmeintegration im Verfahren selbst genutzt werden, insbesondere zur Aufheizung durch indirekte Wärmeübertragung auf den aus dem Acetylene-Ausgaser abgezogenen Sumpfstrom und/oder auf die Flüssigkeit, die man von einer oder mehreren Trennstufen der Trennwandkolonne abzieht, durch indirekten Wärmetausch mit dem heißen Sumpfstrom erwärmt und/oder verdampft und erneut in den unteren gemeinsamen Kolonnenbereich der Trennwandkolonne zurückführt, wobei man die Trennstufe (n) vorteilhaft in der Weise wählt, dass der Gesamtenergiebedarf für die Extraktivdestillationskolonne minimal ist.

Zusätzlich oder alternativ kann der Wärmeinhalt des Sumpfstromes des gereinigten Lösungsmittels aus dem Acetylene-Ausgaser zur indirekten Wärmeübertragung auf die Flüssigkeit genutzt werden, die man von einer oder mehreren geeigneten Trennstufen aus dem unteren gemeinsamen Kolonnenbereich der Extraktivdestillationskolonne abzieht, die Flüssigkeit erwärmt und/oder verdampft und erneut der Extraktivdestillationskolonne zuführt und/oder zur Wärmeübertragung durch indirekten Wärmetausch mit dem der Extraktivdestillationskolonne zuzuführenden C₄-Schnitt.

Es wurde gefunden, dass die Wärmeintegration im vorliegenden Verfahren aufgrund des wesentlich steiler abfallenden Wärmeprofils in der Extraktivdestillationskolonne, vom Sumpfverdampfer über die untersten Trennstufen der Extraktivdestillationskolonne betrachtet, gegenüber bekannten Verfahren, insbesondere gegenüber dem Verfahren der DE-A 103 22 655, insbesondere um etwa 10 % gegenüber dem genannten Verfahren, günstiger ist.

Durch die besondere Betriebsweise der Extraktivdestillationskolonne, in dem die C₄-Acetyleneabtrennung in einem hiervon getrennten Apparat oder Apparateteil durchgeführt wird, wird im vorliegenden Verfahren eine erhöhte Betriebssicherheit gewährleistet, da die Gefahr der Acetylene-Akkumulation über deren Zerfallsgrenze hinaus ausgeschlossen wird.

Darüber hinaus wird durch die besondere Betriebsweise ein überraschend vorteilhaftes Temperaturprofil in der Trennwandkolonne erreicht: obwohl der Sumpfverdampfer der Trennwandkolonne nur bei gegenüber dem bekannten Verfahren geringfügig abgesenkter Temperatur betrieben wird, wird im vorliegenden Verfahren in allen Kolonnen, insbesondere auch in der Trennwandkolonne und dem Acetylene-Ausgaser keine für das Fouling kritische Temperatur, d.h. keine Temperatur von in der Regel > 150 °C erreicht. Die Temperatur wird im Gegenteil in dem Bereich, in dem die Acetylene ausgegast werden, deutlich, um etwa 30-40 °C, gegenüber dem bekannten Verfahren abgesenkt, insbesondere mit der Folge, dass dadurch wesentlich weniger Fouling entsteht.

Die Erfindung wird im Folgenden anhand einer Zeichnung und eines Ausführungsbeispiels näher erläutert:

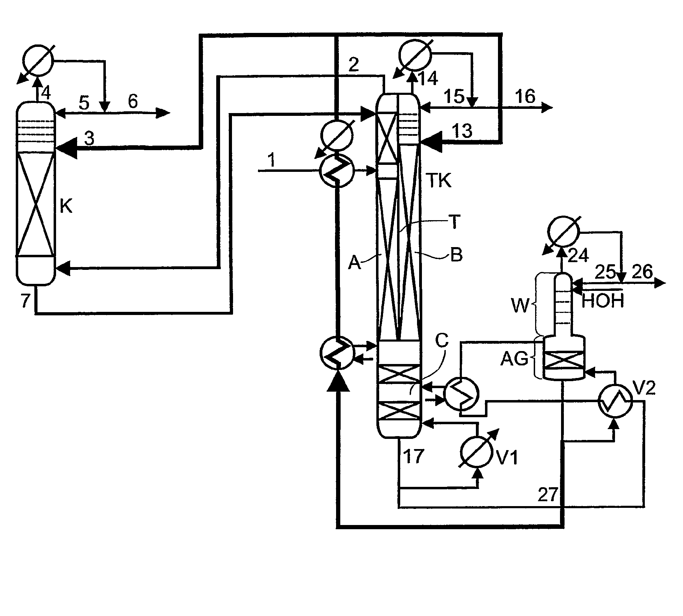

Figur 1 zeigt das Schema einer Anlage nach der Erfindung.

In einer Trennwandkolonne TK mit einer in Kolonnenlängsrichtung angeordneten Trennwand T, die die Trennwandkolonne in einen ersten Teilbereich A, einen zweiten Teilbereich B und einen unteren gemeinsamen Kolonnenbereich C aufteilt, wird dem ersten Teilbereich A ein C₄-Schnitt 1 zugeführt. Beispielhaft enthält der zweite Teilbereich B 40 theoretische Trennstufen und der untere gemeinsame Kolonnenbereich C 10 theoretische Trennstufen. Der Kopfstrom 2 aus dem Teilbereich A wird in den unteren Bereich der vorgeschalteten Extraktivwaschkolonne K mit beispielhaft 20 theoretischen Trennstufen geleitet. Die Extraktivwaschkolonne K wird mit einem ersten Lösungsmittelteilstrom 3, in deren oberen Bereich, beaufschlagt, es findet eine Gegenstromextraktion statt, wobei ein Sumpfstrom 7 anfällt, der zurück in den oberen Bereich des Teilbereichs A der Trennwandkolonne TK geführt wird und ein Kopfstrom 4, der in einem Kondensator am Kopf der Extraktivwaschkolonne K kondensiert wird, wobei ein Teilstrom des Kondensats als Strom 5 erneut auf die Extraktivwaschkolonne K aufgegeben und das Kondensat im übrigen als Strom 6 abgezogen wird.

Die Trennwandkolonne TK wird in deren zweiten Teilbereich B mit einem zweiten Lösungsmittelteilstrom 13 beaufschlagt. Aus dem zweiten Teilbereich B wird ein Kopfstrom 14 abgezogen und kondensiert, ein Teilstrom 15 des kondensierten Kopfstroms 14 als Rücklauf auf den zweiten Teilbereich B der Trennwandkolonne gegeben und der kondensierte Kopfstrom 14 im übrigen als Roh-1,3-Butadien (Strom 16) abgezogen.

Aus dem Sumpf der Trennwandkolonne (TK) wird über den Sumpfverdampfer (V1) der Trennwandkolonne (TK) der Anlage von außen Energie zugeführt. Durch eine geeignete Wärmeintegration innerhalb des Verfahrens wird der Anlage bevorzugt ausschließlich an dieser Stelle Energie von außen zugeführt.

Der Sumpfstrom 17, mit den C₄-Acetylenen beladenes Lösungsmittel, dessen 1,3-Butadien-Anteil eine Obergrenze nicht überschreitet, deren Verlust wirtschaftlich akzeptabel ist, wird, bevorzugt nach Wärmeintegration mit dem Sumpfstrom aus dem Acetylene-Ausgaser (AG) und weiter bevorzugt mit Flüssigkeit, die aus dem unteren gemeinsamen Kolonnenbereich (C) der Trennwandkolonne (TK) abgezogen wird, dem Acetylene-Ausgaser (AG) im oberen Bereich desselben zugeführt. Im Acetylene-Ausgaser (AG) wird ein Sumpfstrom 27, enthaltend gereinigtes Lösungsmittel, abgezogen, der, wie in der Figur dargestellt, bevorzugt nach Wärmeintegration mit Flüssigkeit, die aus dem unteren gemeinsamen Kolonnenbereich (C) der Trennwandkolonne abgezogen wird sowie mit dem der Trennwandkolonne zugeführten C₄-Schnitt, Strom 1, als Strom 3 und Strom 13 in das Verfahren rezykliert.

Auf den Acetylene-Ausgaser (AG) ist ein Wasserwäscher aufgesetzt, worin Lösungsmittelreste aus dem ausgegasten Acetylenestrom mit Rücklauf und Frischwasser ausgewaschen werden. Am Kopf des Wasserwäschers (W) wird ein Acetylene enthaltender Strom 24 abgezogen, in einem Kondensator am Kolonnenkopf kondensiert, teilweise als Rücklauf 25 wieder auf den Wasserwäscher (W) aufgegeben und im übrigen als Strom 26 aus dem Verfahren ausgeschleust.

## Patentansprüche

1. Verfahren zur Gewinnung von Roh-1,3-Butadien durch Extraktivdestillation mit einem selektiven Lösungsmittel aus einem C4-Schnitt, enthaltend C4-Acetylene als Nebenkomponenten, in einer Trennwandkolonne (TK) mit einem Sumpfverdampfer (V1), in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet und der eine Extraktivwaschkolonne (K) vorgeschaltet ist, **dadurch gekennzeichnet, dass** man den Energieeintrag in die Trennwandkolonne (TK) über den Sumpfverdampfer (V1) so regelt, dass man aus der Trennwandkolonne (TK) einen Sumpfstrom (17) abzieht, enthaltend mit den C4-Acetylenen beladenes Lösungsmittel, dessen Anteil an 1,3-Butadien auf das maximal 0,1 bis 2-fache des Anteils an C4-Acetylenen so begrenzt ist, dass der Verlust an 1,3-Butadien wirtschaftlich akzeptabel ist, und dass man den Sumpfstrom (17) einem Acetylene-Ausgaser (AG) zuführt und im Acetylene-Ausgaser (AG) die C4-Acetylene über Kopf ausstrippt und gereinigtes Lösungsmittel als Sumpfstrom (27) gewinnt.

2. Verfahren nach Anspruch 1, **dadurch gekennzeichnet, dass** man den Anteil an 1,3-Butadien im Sumpfstrom (17) der Trennwandkolonne (TK) auf das 0,3-fache des Anteils an C4-Acetylenen begrenzt.

3. Verfahren nach Anspruch 1 oder 2, **dadurch gekennzeichnet, dass** man die Energie des Sumpfstroms (17) der Trennwandkolonne (TK) zum indirekten Wärmetausch mit dem Sumpfstrom (27) des Acetylene-Ausgasers und/oder mit der Flüssigkeit nutzt, die man von einer oder mehreren Trennstufen aus dem unteren gemeinsamen Kolonnenbereich C der Trennwandkolonne abzieht, wobei man die Trennstufe, von der man die Flüssigkeit abzieht, dergestalt wählt, dass der Energiebedarf für die Trennwandkolonne (TK) minimal ist.

4. Verfahren nach einem der Ansprüche 1-3, **dadurch gekennzeichnet, dass** man den Wärmeinhalt des Sumpfstroms (27) des Acetylene-Ausgasers (AG) zum indirekten Wärmetausch mit der Flüssigkeit nutzt, die man von einer oder mehreren Trennstufen aus dem unteren gemeinsamen Kolonnenbereich (C) der Trennwandkolonne abzieht, wobei man die Trennstufe (n), von der man die Flüssigkeit abzieht, dergestalt bestimmt, dass der Energiebedarf für die Trennwandkolonne (TK) minimal ist und/oder dass man den Wärmeinhalt des Sumpfstromes (27) zum indirekten Wärmetausch mit dem der Trennwandkolonne (TK) zugeführten aufzutrennenden C4-Schnitt (1) nutzt.

5. Verfahren nach einem der Ansprüche 1-4, **dadurch gekennzeichnet, dass** man anstelle der Trennwandkolonne (TK) thermisch gekoppelte Kolonnen einsetzt.

6. Verfahren nach einem der Ansprüche 1-5, **dadurch gekennzeichnet, dass**
- der C4-Schnitt (1) dem ersten Teilbereich (A) der Trennwandkolonne (TK), bevorzugt in dessen mittleren Bereich, zugeführt wird,
- der Kopfstrom (2) aus dem ersten Teilbereich (A) der Trennwandkolonne (TK) der Extraktivwaschkolonne (K), in deren unteren Bereich, zugeführt wird,
- in der Extraktivwaschkolonne (K) eine Gegenstromextraktion durch Beaufschlagung mit einem ersten Teilstrom (3) des selektiven Lösungsmittels im oberen Bereich der Extraktivwaschkolonne (K) durchgeführt wird,
- die im selektiven Lösungsmittel weniger als 1,3-Butadien löslichen Komponenten des C4-Schnittes über Kopf der Extraktivwaschkolonne (K) abgezogen (4) werden;
- der Sumpfstrom (7) aus der Extraktivwaschkolonne (K) in den oberen Bereich des ersten Teilbereichs (A) der Trennwandkolonne (TK) zurückgeführt wird,
- der Trennwandkolonne (TK) im oberen Bereich des zweiten Teilbereichs (B) ein zweiter Teilstrom (13) des selektiven Lösungsmittels zugeführt wird,
- das Kopfprodukt (14) aus dem zweiten Teilbereich (B) der Trennwandkolonne (TK) als Roh-1,3-Butadien (16) abgezogen wird und
- aus dem unteren gemeinsamen Kolonnenbereich (C) der Trennwandkolonne (TK) ein Sumpfstrom (17), bestehend aus mit den C4-Acetylenen beladenem Lösungsmittel, dessen Anteil an 1,3-Butadien so begrenzt ist, dass der Verlust an 1,3-Butadien wirtschaftlich akzeptabel ist, abgezogen wird,
- der Sumpfstrom (17) dem Acetylene-Ausgaser (AG) zugeführt wird, worin die C4-Acetylene über Kopf ausgestrippt und gereinigtes Lösungsmittel als Sumpfstrom (27) gewonnen wird, der in das Verfahren rezykliert wird.

7. Verfahren nach einem der Ansprüche 1-6, **dadurch gekennzeichnet, dass** man die Temperatur im Sumpfverdampfer (V1) der Trennwandkolonne (TK) auf einen Wert im Bereich von 150 bis 210°C, bevorzugt auf 178 °C und den Kopfdruck des zweiten Teilbereichs (B) der Trennwandkolonne (TK) auf einen Wert im Bereich von 1 bis 10 bar absolut, bevorzugt auf einen Wert im Bereich von 2 bis 5 bar absolut, besonders bevorzugt auf 3,5 bar absolut und dem Kopfdruck im Acetylene-Ausgaser (AG) auf einen Wert im Bereich von 1 bar absolut bis maximal dem Sumpfdruck in der Trennwandkolonne (TK), regelt.

8. Verfahren nach einem der Ansprüche 1-7, **dadurch gekennzeichnet, dass** man den Acetylene-Ausgaser (AG) konstruktiv in den unteren gemeinsamen Kolonnenbereich (C) integriert, indem man die Zahl der theoretischen Trennstufen im unteren gemeinsamen Kolonnenbereich (C) entsprechend größer auslegt und in der Trennwandkolonne (TK) an der Stelle, die dem oberen Ende des in den unteren gemeinsamen Kolonnenbereich (C) integrierten Acetylene-Ausgaser (AG) entspricht, eine gasdichte Abtrennung einbaut.

## Claims

1. A process for obtaining crude 1,3-butadiene by extractive distillation with a selective solvent from a C4 cut comprising C4 acetylenes as secondary components in a dividing wall column (TK) having a bottom evaporator (V1), in which a dividing wall (T) is disposed in the longitudinal direction of the column to form a first subregion (A), a second subregion (B) and a lower combined column region (C), and which is disposed upstream of an extractive wash column (K), which comprises controlling the energy input into the dividing wall column (TK) via the bottom evaporator (V1) in such a way that a bottom stream (17) is drawn off from the dividing wall column (TK) and comprises solvent laden with the C4 acetylenes whose proportion of 1,3-butadiene is restricted to a maximum of from 0.1 to 2 times the proportion of C4 acetylenes such that the loss of 1,3-butadiene is economically acceptable, and feeding the bottom stream (17) to an acetylenes outgasser (AG) and, in the acetylenes outgasser (AG), stripping out the C4 acetylenes overhead and obtaining purified solvent as the bottom stream (27).

2. The process according to claim 1, wherein the proportion of 1,3-butadiene in the bottom stream (17) of the dividing wall column (TK) is restricted to 0.3 times the proportion of C4 acetylenes.

3. The process according to claim 1 or 2, wherein the energy of the bottom stream (17) of the dividing wall column (TK) is utilized for indirect heat exchange with the bottom stream (27) of the acetylenes degasser and/or with the liquid which is drawn off from one or more separation stages in the lower combined column region C of the dividing wall column, and the separation stage from which the liquid is drawn off is selected in such a way that the energy demand for the dividing wall column (TK) is minimal.

4. The process according to any of claims 1-3, wherein the heat content of the bottom stream (27) of the acetylenes outgasser (AG) is utilized for indirect heat exchange with the liquid which is drawn off from one or more separation stages in the lower combined column region (C) of the dividing wall column, and the separation stage(s) from which the liquid is drawn off is/are determined in such a way that the energy demand for the dividing wall column (TK) is minimal, and/or that the heat content of the bottom stream (27) is utilized for indirect heat exchange with the C4 cut (1) to be separated which is fed to the dividing wall column (TK).

5. The process according to any of claims 1-4, wherein thermally coupled columns are used instead of the dividing wall column (TK).

6. The process according to any of claims 1-5, wherein
- the C4 cut (1) is fed to the first subregion (A) of the dividing wall column (TK), preferably into its middle region,
- the top stream (2) from the first subregion (A) of the dividing wall column (TK) is fed to the extractive wash column (K), into its lower region,
- in the extractive wash column (K), a countercurrent extraction is carried out by charging with a first substream (3) of the selective solvent in the upper region of the extractive wash column (K),
- the components of the C4 cut having lower solubility than 1,3-butadiene in the selective solvent are drawn off (4) via the top of the extractive wash column (K),
- the bottom stream (7) from the extractive wash column (K) is recycled into the upper region of the first subregion (A) of the dividing wall column (TK),
- a second substream (13) of the selective solvent is fed to the dividing wall column (TK) in the upper region of the second subregion (B),
- the top product (14) from the second subregion (B) of the dividing wall column (TK) is drawn off as crude 1,3-butadiene (16) and
- a bottom stream (17) consisting of solvent laden with the C4 acetylenes, whose proportion of 1,3-butadiene is restricted such that the loss of 1,3-butadiene is economically acceptable, is drawn off from the lower combined column region (C) of the dividing wall column (TK),
- the bottom stream (17) is fed to the acetylenes degasser (AG) in which the C4 acetylenes are stripped out overhead and purified solvent is obtained as the bottom stream (27) and is recycled into the process.

7. The process according to any of claims 1-6, wherein the temperature in the bottom evaporator (V1) of the dividing wall column (TK) is controlled to a value in the range from 50 to 210°C, preferably to 178°C, and the top pressure of the second subregion (B) of the dividing wall column (TK) to a value in the range from 1 to 10 bar absolute, preferably to a value in the range from 2 to 5 bar absolute, more preferably to 3.5 bar absolute, and the top pressure in the acetylenes outgasser (AG) to a value in the range from 1 bar absolute to a maximum of the bottom pressure in the dividing wall column (TK).

8. The process according to any of claims 1-7, wherein the acetylenes outgasser (AG) is integrated by construction into the lower combined column region (C) by configuring the number of theoretical plates in the lower combined column region (C) to a correspondingly larger value and incorporating a gas-tight division in the dividing wall column (TK) at the point which corresponds to the upper end of the acetylenes outgasser (AG) integrated into the lower combined column region (C).

## Revendications

1. Procédé d'obtention de 1,3-butadiène brut par distillation extractive avec un solvant sélectif à partir d'une coupe en C4 contenant des acétylènes en C4 en tant que composants secondaires, dans une colonne à paroi de séparation (TK) comprenant un évaporateur de fond (V1), dans laquelle une paroi de séparation (T) est placée dans la direction longitudinale de la colonne en formant une première zone (A), une seconde zone (B) et une zone de colonne commune inférieure (C), et à laquelle une colonne de lavage extractive (K) est connectée en amont, **caractérisé en ce que** l'apport d'énergie dans la colonne à paroi de séparation (TK) par l'évaporateur de fond (V1) est régulé de manière à soutirer de la colonne à paroi de séparation (TK) un courant de fond (17) contenant le solvant chargé avec les acétylènes en C4, dont la proportion de 1,3-butadiène est limitée au plus à 0,1 à 2 fois la proportion d'acétylènes en C4, de manière à ce que la perte en 1,3-butadiène soit acceptable économiquement, et à introduire le courant de fond (17) dans un dégazeur d'acétylènes (AG) et extraire les acétylènes en C4 par la tête dans le dégazeur d'acétylènes (AG) et obtenir le solvant purifié en tant que courant de fond (27).

2. Procédé selon la revendication 1, **caractérisé en ce que** la proportion de 1,3-butadiène dans le courant de fond (17) de la colonne à paroi de séparation (TK) est limitée à 0,3 fois la proportion d'acétylènes en C4.

3. Procédé selon la revendication 1 ou 2, **caractérisé en ce que** l'énergie du courant de fond (17) de la colonne à paroi de séparation (TK) est utilisée pour l'échange de chaleur indirect avec le courant de fond (27) du dégazeur d'acétylènes et/ou avec le liquide soutiré d'une ou de plusieurs étapes de séparation de la zone de colonne commune inférieure C de la colonne à paroi de séparation, l'étape de séparation à partir de laquelle le liquide est soutiré étant choisie de manière à ce que la demande énergétique pour la colonne à paroi de séparation (TK) soit minimale.

4. Procédé selon l'une quelconque des revendications 1 à 3, **caractérisé en ce que** la capacité calorifique du courant de fond (27) du dégazeur d'acétylènes (AG) est utilisée pour l'échange de chaleur indirect avec le liquide soutiré d'une ou de plusieurs étapes de séparation de la zone de colonne commune inférieure (C) de la colonne à paroi de séparation, la ou les étapes de séparation à partir desquelles le liquide est soutiré étant déterminées de manière à ce que la demande énergétique pour la colonne à paroi de séparation (TK) soit minimale et/ou **en ce que** la capacité calorifique du courant de fond (27) est utilisée pour l'échange de chaleur indirect avec la coupe en C4 (1) à séparer introduite dans la colonne à paroi de séparation (TK).

5. Procédé selon l'une quelconque des revendications 1 à 4, **caractérisé en ce que** des colonnes couplées thermiquement sont utilisées à la place de la colonne à paroi de séparation (TK).

6. Procédé selon l'une quelconque des revendications 1 à 5, **caractérisé en ce que**
- la coupe en C4 (1) est introduite dans la première zone (A) de la colonne à paroi de séparation (TK), de préférence dans sa partie centrale,
- le courant de tête (2) de la première zone (A) de la colonne à paroi de séparation (TK) est introduit dans la colonne de lavage extractive (K), dans sa partie inférieure,
- dans la colonne de lavage extractive (K), une extraction à contre-courant est réalisée par injection d'un premier courant partiel (3) du solvant sélectif dans la partie supérieure de la colonne de lavage extractive (K),
- les composants de la coupe en C4 moins solubles que le 1,3-butadiène dans le solvant sélectif sont soutirés (4) par la tête de la colonne de lavage extractive (K),
- le courant de fond (7) de la colonne de lavage extractive (K) est recyclé dans la partie supérieure de la première zone (A) de la colonne à paroi de séparation (TK),
- un second courant partiel (13) du solvant sélectif est introduit dans la colonne à paroi de séparation (TK) dans la partie supérieure de la seconde zone (B),
- le produit de tête (14) de la seconde zone (B) de la colonne à paroi de séparation (TK) est soutiré en tant que 1,3-butadiène brut (16) et
- un courant de fond (17) constitué du solvant chargé avec les acétylènes en C4, dont la proportion de 1,3-butadiène est limitée de manière à ce que la perte en 1,3-butadiène soit acceptable économiquement, est soutiré de la zone de colonne commune inférieure (C) de la colonne à paroi de séparation (TK),
- le courant de fond (17) est introduit dans le dégazeur d'acétylènes (AG), dans lequel les acétylènes en C4 sont extraits par la tête et le solvant purifié est obtenu en tant que courant de fond (27), qui est recyclé dans le procédé.

7. Procédé selon l'une quelconque des revendications 1 à 6, **caractérisé en ce que** la température dans l'évaporateur de fond (V1) de la colonne à paroi de séparation (TK) est régulée à une valeur dans la plage allant de 150 à 210 °C, de préférence à 178 °C, et la pression de tête de la seconde zone (B) de la colonne à paroi de séparation (TK) à une valeur dans la plage allant de 1 à 10 bar absolu, de préférence à une valeur dans la plage allant de 2 à 5 bar absolu, de manière particulièrement préférée à 3,5 bar absolu, et la pression de tête dans le dégazeur d'acétylènes (AG) à une valeur dans la plage allant de 1 bar absolu jusqu'à au plus la pression de fond de la colonne à paroi de séparation (TK) .

8. Procédé selon l'une quelconque des revendications 1 à 7, **caractérisé en ce que** le dégazeur d'acétylènes (AG) est intégré par construction dans la zone de colonne commune inférieure (C) en augmentant en conséquence le nombre d'étapes de séparation théoriques dans la zone de colonne commune inférieure (C) et en incorporant une séparation étanche aux gaz dans la colonne à paroi de séparation (TK) à l'emplacement qui correspond à l'extrémité supérieure du dégazeur d'acétylènes (AG) intégré dans la zone de colonne commune inférieure (C).
